# FASCICULE DE BREVET EUROPEEN

(11) **EP 3 201 832 B1**
(45) Date de publication et mention de la délivrance du brevet: **10.10.2018**
(21) Numéro de dépôt: 15788117.8
(22) Date de dépôt: 30.09.2015
(51) Int. Cl.: G06K 9/00, H04N 5/3745, H04N 5/33

(54) **CAPTEUR D'EMPREINTES DIGITALES OU PALMAIRES**
FINGERABDRUCK- ODER HANDFLÄCHENSENSOR
FINGERPRINT OR PALMPRINT SENSOR

(30) Priorité: 03.10.2014 FR 1459494
(43) Date de publication de la demande: 09.08.2017
(73) Titulaire: Commissariat à l'Energie Atomique et aux Energies Alternatives, 75015 Paris (FR); Safran Identity & Security, 92130 Issy-les-Moulineaux (FR)
(72) Inventeur: MAINGUET, Jean-François, 38100 Grenoble (FR); FOURRE, Joel Yann, 78160 Marly-le-Roi (FR); SEGURA PUCHADES, Josep, 38600 Fontaine (FR)
(74) Mandataire: Cabinet Beaumont
(86) Numéro de dépôt international: PCT/FR2015/052616
(87) Numéro de publication internationale: WO 2016/051087

(56) Documents cités:
- EP-A1- 0 977 275
- WO-A1-2012/158950
- WO-A1-2014/091093
- JP-A- 2006 343 229
- "Chapter 2: Fingerprint Sensing" In: Davide Maltoni; Dario Maio; Anil K Jain; Salil Prabhakar: "Handbook of Fingerprint Recognition", 1 janvier 2009 (2009-01-01), SPRINGER, LONDON, GB, XP002714829, ISBN: 978-1-84882-253-5 pages 57-95, 2.3 Live-Scan Fingerprint Sensing
- Jean-François Mainguet: "Biometrics: fingerprint sensing techniques", , 27 septembre 2014 (2014-09-27), XP055192301, Extrait de l'Internet: URL:http://web.archive.org/web/20140927104 016/http://fingerchip.pagesperso-orange.fr /biometrics/types/fingerprint_sensors_phys ics.htm [extrait le 2015-05-29]
- HIRO HAN ET AL: "Characteristics of thermal-type fingerprint sensor", PROCEEDINGS OF SPIE, vol. 6944, 16 mars 2008 (2008-03-16), page 69440P, XP055192306, ISSN: 0277-786X, DOI: 10.1117/12.777024

## Description

La présente demande de brevet revendique la priorité de la demande de brevet français FR14/59494 qui sera considérée comme faisant partie intégrante de la présente description.

### Domaine

La présente demande concerne le domaine des capteurs d'empreintes digitales ou palmaires.

### Exposé de l'art antérieur

Divers types de capteurs ont été proposés pour réaliser une acquisition électronique d'une empreinte digitale et/ou palmaire, c'est-à-dire pour fournir une image du motif formé par les crêtes et creux de la peau d'un doigt, de plusieurs doigts, et/ou de la paume de la main. On a notamment proposé des capteurs optiques, des capteurs capacitifs, des capteurs thermiques, des capteurs à ultrasons, et des capteurs à champ électrique. De tels capteurs sont divulgués dans les documents WO2014/091093 ou JP2006343229.

On s'intéresse ici plus particulièrement aux capteurs d'empreintes réalisés en technologie TFT (de l'anglais "Thin Film Transistor" - transistor en couches minces), c'est-à-dire comportant, sur un substrat de support, une ou plusieurs cellules élémentaires d'acquisition, chaque cellule élémentaire (ou pixel) comportant un élément d'acquisition, par exemple photoélectrique, pyroélectrique ou capacitif, et un ou plusieurs transistors TFT permettant de commander cet élément. Par transistor TFT, on entend ici des transistors formés par dépôts successifs de couches conductrices, isolantes et semiconductrices sur le substrat de support. En particulier, dans un transistor TFT, la région semiconductrice de formation de canal du transistor est réalisée par dépôt d'une couche d'un matériau semiconducteur, par exemple du silicium amorphe hydrogéné, du silicium polycristallin (rendu polycristallin après un recuit par exemple), ou encore un matériau de type IGZO (de l'anglais "Indium Gallium Zinc Oxyde" - oxyde d'indium gallium zinc), ce dépôt pouvant être précédé d'un dépôt d'une couche conductrice servant à former une électrode de grille, de source ou de drain du transistor. Les capteurs d'empreintes réalisés en technologie TFT présentent l'avantage d'être relativement peu onéreux, notamment grâce à l'utilisation d'un substrat de support en un matériau à faible coût tel que le verre (au lieu d'un substrat en silicium monocristallin généralement utilisé pour réaliser des transistors), et d'être facilement intégrables dans de nombreux types de dispositifs électroniques, et en particulier dans des dispositifs utilisant déjà la technologie TFT pour réaliser d'autres fonctions, par exemple pour réaliser des écrans d'affichage. La technologie TFT est tout particulièrement avantageuse dans le domaine des capteurs d'empreintes dans lesquels la surface du capteur est sensiblement identique à la surface de l'empreinte à acquérir, c'est-à-dire dans lesquels aucun système optique de focalisation (ou objectif) n'est placé entre le capteur et l'objet dont on souhaite acquérir une image. En effet, en raison de leurs grandes tailles, la réalisation de tels capteurs dans et sur des substrats de silicium aurait un coût bien trop élevé pour la plupart des applications.

### Résumé

Un mode de réalisation prévoit un capteur d'empreintes digitales ou palmaires comportant : sur un substrat de support, une pluralité de cellules élémentaires d'acquisition, réalisées en technologie TFT, chaque cellule comportant un photodétecteur et un élément de conversion pyroélectrique reliés à un même noeud capacitif de lecture de la cellule, et un circuit de lecture reliant le noeud capacitif de lecture de la cellule à une piste conductrice de sortie de la cellule une source lumineuse d'éclairage ; et une source de chaleur distincte de la source lumineuse.

Selon un mode de réalisation, le substrat de support est transparent.

Selon un mode de réalisation, le photodétecteur et l'élément de conversion pyroélectrique sont connectés en parallèle.

Selon un mode de réalisation, le capteur comporte un circuit adapté à commander l'acquisition d'une image thermique et l'acquisition d'une image optique par les cellules du capteur, ce circuit étant adapté, pendant toute la phase d'acquisition de l'image optique, à allumer la source lumineuse et maintenir la source de chaleur éteinte, et, pendant toute la phase d'acquisition de l'image thermique, à maintenir la source lumineuse éteinte.

Selon un mode de réalisation, le capteur comporte un circuit adapté à commander l'acquisition d'une image thermique et l'acquisition d'une image optique par les cellules du capteur, ce circuit étant adapté à mettre en oeuvre l'acquisition de l'image thermique pendant une partie d'une période d'intégration de la phase d'acquisition de l'image optique pendant laquelle la source lumineuse est allumée.

Selon un mode de réalisation, dans chaque cellule, le photodétecteur et l'élément de conversion pyroélectrique sont connectés au à un noeud capacitif de lecture de la cellule.

Selon un mode de réalisation, dans chaque cellule, le photodétecteur et l'élément de conversion pyroélectrique sont connectés à un noeud intermédiaire de la cellule, le noeud intermédiaire étant relié au noeud capacitif de lecture de la cellule par un transistor de sélection, et chaque cellule comprenant en outre : un condensateur de référence connecté entre le noeud capacitif de lecture et un noeud d'application d'un signal de commande ; et une électrode connectée au noeud capacitif de lecture, l'électrode étant revêtue d'une couche diélectrique et étant destinée à former une capacité avec la peau d'un utilisateur en vue d'une acquisition capacitive d'une image d'empreinte.

Selon un mode de réalisation, le photodétecteur et l'élément de conversion pyroélectrique sont connectés à un même transistor de sélection permettant, à l'état ouvert, d'isoler le photodétecteur de l'élément pyroélectrique.

Selon un mode de réalisation, les cellules sont disposées en matrice selon des lignes et des colonnes, et la source de chaleur est commandable pour chauffer les cellules ligne par ligne.

Selon un mode de réalisation, les cellules sont des cellules à lecture en tension, chaque cellule comportant : un transistor de réinitialisation reliant le noeud capacitif de lecture à un noeud d'application d'un potentiel de réinitialisation et le un circuit de lecture de chaque cellule comportant un transistor monté en source suiveuse, dont la grille est connectée au noeud de lecture, et dont la source est reliée à une piste de sortie de la cellule par l'intermédiaire d'un transistor de lecture.

Selon un mode de réalisation, les cellules sont des cellules à lecture en charge, le circuit de lecture de chaque cellule comportant un transistor de lecture reliant le noeud de lecture à une piste de sortie de la cellule.

### Brève description des dessins

Ces caractéristiques et leurs avantages, ainsi que d'autres, seront exposés en détail dans la description suivante de modes de réalisation particuliers faite à titre non limitatif en relation avec les figures jointes parmi lesquelles :
la figure 1 est un schéma électrique illustrant un exemple d'un capteur d'empreintes TFT optique ;
la figure 2 est un schéma électrique illustrant un exemple d'un capteur d'empreintes TFT thermique ;
la figure 3 est un schéma électrique illustrant un autre exemple d'un capteur d'empreintes TFT optique ou thermique ;
la figure 4 est un schéma électrique illustrant un exemple d'un capteur d'empreintes TFT capacitif ;
la figure 5 est un schéma électrique illustrant un autre exemple de réalisation d'un capteur d'empreintes TFT ;
la figure 6 est un schéma électrique illustrant un premier mode de réalisation d'un capteur d'empreintes TFT ;
la figure 7 est un schéma électrique illustrant une variante de réalisation du capteur de la figure 6 ;
la figure 8 est un schéma électrique illustrant une autre variante de réalisation du capteur de la figure 6 ;
la figure 9 est un schéma électrique illustrant un deuxième mode de réalisation d'un capteur d'empreintes TFT ; et
la figure 10 est un schéma électrique illustrant une variante de réalisation du capteur de la figure 9.

### Description détaillée

Par souci de clarté, de mêmes éléments ont été désignés par de mêmes références aux différentes figures. De plus, seuls les éléments utiles à la compréhension des modes de réalisation décrits ont été détaillés. En particulier, les circuits périphériques de commande des cellules élémentaires des capteurs d'empreintes TFT décrits n'ont pas été détaillés, la réalisation de tels circuits étant à la portée de l'homme de l'art à la lecture de la présente description. On notera par ailleurs que dans la présente description, lorsque des architectures de cellules élémentaires, de matrices de cellules élémentaires ou de capteurs d'empreintes sont décrites, on utilise le terme "connecté" pour désigner une liaison électrique directe, sans composant électronique intermédiaire, par exemple au moyen d'une piste conductrice, et le terme "couplé" ou le terme "relié", pour désigner une liaison électrique directe ou via un ou plusieurs composants intermédiaires, par exemple via un transistor.

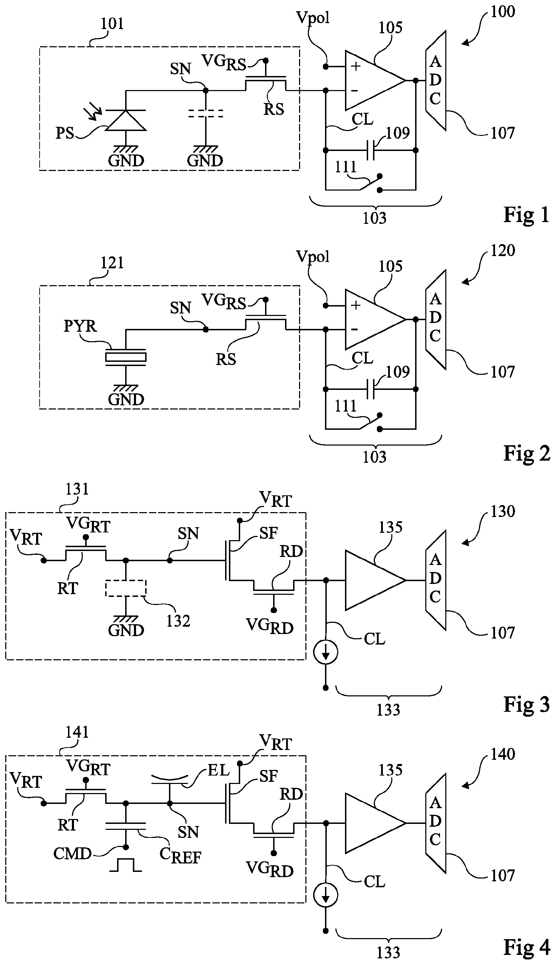

La figure 1 est un schéma électrique illustrant un exemple d'un capteur d'empreintes TFT optique 100. Le capteur 100 comprend une pluralité de cellules élémentaires d'acquisition 101 identiques ou similaires, réalisées en technologie TFT sur une face d'un substrat de support transparent, par exemple en verre, que l'on appellera ci-après par convention la face supérieure du substrat. Par souci de simplification, une seule cellule 101 a été représentée sur la figure 1. Chaque cellule 101 comprend un photodétecteur PS, par exemple une photodiode dont l'anode est reliée à un noeud d'application d'un potentiel de référence GND (par exemple la masse), et dont la cathode est connectée à un noeud capacitif de lecture SN de la cellule. A titre d'exemple, le photodétecteur PS est une photodiode de type PIN, ou une photodiode organique. Sur la figure 1, la capacité du noeud de lecture SN a été représentée en traits pointillés sous la forme d'un condensateur dont une électrode est connectée au noeud SN et dont l'autre électrode est connectée au noeud GND. En pratique, la capacité du noeud SN peut être une capacité parasite d'un autre élément de la cellule, par exemple la capacité parasite de la photodiode PS (à laquelle s'additionne la capacité parasite du transistor RS dans l'exemple représenté), ou une capacité spécifique. Par souci de simplification, la capacité du noeud SN ne sera pas représentée sur les figures suivantes. Chaque cellule 101 comprend en outre un transistor de lecture RS reliant son noeud de lecture SN à une piste conductrice de sortie CL de la cellule. La grille de commande du transistor RS est connectée à un noeud VG_{RS} d'application d'un potentiel de commande de ce transistor. La piste de sortie CL de la cellule 101 est connectée à un étage de sortie 103 du capteur. Dans cet exemple, l'étage de sortie 103 comprend un amplificateur opérationnel 105 dont une entrée inverseuse (-) reliée à la piste CL et dont une entrée non inverseuse (+) reliée à un noeud Vₚₒₗ d'application d'un potentiel de polarisation. L'étage de sortie 103 comprend en outre un convertisseur analogique-numérique 107 (ADC) dont l'entrée est reliée à la sortie de l'amplificateur 105. Dans l'exemple représenté, l'étage de sortie comprend en outre un condensateur 109 en parallèle avec un interrupteur de commande 111 entre l'entrée inverseuse (-) et la sortie de l'amplificateur 105.

Le capteur 100 comprend en outre une source lumineuse d'éclairage, non représentée. A titre d'exemple, la source d'éclairage est disposée du côté de la face du substrat opposée aux cellules 101, que l'on appellera ci-après par convention la face inférieure du substrat.

Le fonctionnement du capteur 100 est le suivant. L'utilisateur place un doigt (ou plusieurs) sur ou au-dessus de la surface supérieure du capteur (côté cellules 101). La source lumineuse de rétroéclairage, disposée du côté du substrat opposé aux cellules, éclaire le doigt à travers des zones transparentes de l'ensemble formé par le substrat de support et les cellules 101. La lumière est alors rétrodiffusée par le doigt en direction des photodiodes PS, avec, au niveau de chaque cellule 101, une atténuation plus ou moins importante selon que la portion de doigt située au-dessus de la cellule correspond à une crête ou à un creux de la peau du doigt. A titre de variante, la source lumineuse peut être placée au-dessus ou à côté du doigt, la lumière étant alors transmise par le doigt en direction des photodiodes PS du capteur, avec une atténuation plus ou moins importante selon que la portion de doigt située au-dessus de la cellule correspond à une crête ou à un creux de la peau du doigt. Dans ce cas, le substrat peut être non transparent. Pendant une période d'intégration d'une cellule 101, le transistor de lecture RS de la cellule est non passant, et la lumière réfléchie par le doigt est convertie en charges électriques par la photodiode PS de la cellule. Ces charges sont stockées sur le noeud de lecture SN de la cellule. A la fin de la période d'intégration, les charges photogénérées sont transférées sur la piste de sortie CL de la cellule (qui peut avoir été préalablement réinitialisée par fermeture de l'interrupteur 111) par l'intermédiaire du transistor RS, et le signal résultant est lu par l'étage de sortie 103 connecté à la piste CL. Le transistor RS étant bidirectionnel, il permet également de réinitialiser la photodiode PS à un potentiel fixé avant le début d'une nouvelle période d'intégration.

A titre d'exemple, plusieurs cellules élémentaires 101 peuvent être connectées à une même piste de sortie CL et partager un même étage de sortie 103 du capteur. Les cellules 101 sont par exemple disposées en matrice selon des lignes et des colonnes, les cellules d'une même colonne étant connectées à une même piste de sortie CL et à un même étage de sortie 103, et les cellules de colonnes distinctes étant connectées à des pistes de sortie CL distinctes et à des étages de sortie 103 distincts. A titre d'exemple, les cellules 101 sont commandables simultanément ligne par ligne, c'est-à-dire que les cellules 101 d'une même ligne ont leurs noeuds VG_{RS} connectés à une même piste de commande et les cellules 101 de lignes distinctes ont leurs noeuds VG_{RS} connectés à des pistes de commande distinctes.

La figure 2 est un schéma électrique illustrant un exemple d'un capteur d'empreintes TFT thermique 120. Le capteur 120 de la figure 2 comprend des éléments communs avec le capteur 100 de la figure 1. Ces éléments ne seront pas décrits à nouveau. Le capteur 120 diffère du capteur 100 principalement en ce que, dans le capteur 120, des cellules élémentaires d'acquisition 121 de type thermique remplacent les cellules élémentaires d'acquisition optiques 101 du capteur 100. Les cellules élémentaires 121 du capteur de la figure 2 diffèrent des cellules élémentaires 101 du capteur de la figure 1 en ce que, dans chaque cellule 121, un élément de conversion pyroélectrique PYR, reliant le noeud de lecture SN de la cellule au noeud GND, remplace le photodétecteur PS des cellules 101. L'élément de conversion pyroélectrique PYR comprend typiquement une couche d'un matériau pyroélectrique tel que le nitrure d'aluminium (AlN), l'oxyde de zinc (ZnO), un polymère tel que le polyfluorure de vinylidène (PVDF), dont le coefficient pyroélectrique est de l'ordre de 40 µC/m²/K, un matériau céramique de type PZT (titanozirconate de plomb), dont le coefficient pyroélectrique est de l'ordre de 350 µC/m²/K, ou un matériau cristallin de type TGS (Triglycine sulfate) ou LiTaO₃, disposée entre deux couches conductrices connectées respectivement au noeud SN et au noeud GND.

Le capteur 120 diffère en outre du capteur 100 de la figure 1 en ce qu'il ne comprend pas de source lumineuse de rétroéclairage, mais comprend une source de chaleur, non représentée. A titre d'exemple la source de chaleur peut comprendre un réseau de résistances régulièrement réparties sur la surface du capteur. A titre d'exemple, la source de chaleur comprend une résistance par cellule élémentaire, cette résistance étant disposée au voisinage de l'élément pyroélectrique de la cellule. Les résistances de la source de chaleur sont de préférence disposées du même côté du substrat de support que les cellules 101, c'est-à-dire du côté de la face supérieure du substrat.

Le fonctionnement du capteur 120 est le suivant. L'utilisateur a placé un doigt (ou plusieurs) sur ou au-dessus de la surface supérieure du capteur (côté cellules 121). La source de chaleur du capteur est alors mise en marche, et chauffe les éléments de conversion pyroélectrique PYR qui génèrent en conséquence des charges électriques sur les noeuds de lecture SN des cellules 121 correspondantes. La quantité de chaleur reçue par chaque élément de conversion pyroélectrique PYR lorsque la source de chaleur est allumée est plus importante lorsque la cellule correspondante est surmontée par un creux de la peau, que lorsqu'elle est surmontée par une crête. En effet, lorsque la cellule est surmontée par une crête, la peau absorbe une part plus importante de la chaleur émise par la source que lorsque la cellule est surmontée par un creux. Ainsi, lorsqu'une cellule 121 est surmontée par un creux de la peau, la quantité de charges électriques générées sur son noeud de lecture SN est plus importante que lorsque la cellule est surmontée par une crête. A la fin d'une période d'intégration pendant laquelle le transistor de lecture RS de la cellule est maintenu non passant, les charges accumulées sur le noeud SN sont transférées sur la piste de sortie CL de la cellule par l'intermédiaire du transistor RS, et le signal résultant est lu par l'étage de sortie 103 connecté à la piste CL. Le transistor RS étant bidirectionnel, il permet également de réinitialiser le noeud SN avant le début d'une nouvelle période d'intégration.

De préférence, lors d'une acquisition, la source de chaleur est commandée pour produire une impulsion de chaleur, et les cellules sont lues un certain temps après le début de l'impulsion, et/ou peu de temps après la fin de cette impulsion, de façon à s'affranchir des phénomènes de thermalisation entraînant, avec le temps, l'uniformisation des niveaux de charge accumulés sur les noeuds de lecture SN des différentes cellules.

Comme dans l'exemple de la figure 1, plusieurs cellules élémentaires 121 peuvent être connectées à une même piste de sortie CL et partager un même étage de sortie 103 du capteur. Les cellules 121 sont par exemple disposées en matrice selon des lignes et des colonnes, les cellules d'une même colonne étant connectées à une même piste de sortie CL et à un même étage de sortie 103, et les cellules de colonnes distinctes étant connectées à des pistes de sortie CL distinctes et à des étages de sortie 103 distincts. A titre d'exemple, les cellules 121 sont commandables simultanément ligne par ligne. De préférence, la source de chaleur est alors commandable pour chauffer les cellules 121 ligne par ligne. Ceci permet de réaliser un balayage du capteur ligne par ligne en synchronisant l'allumage de la source de chaleur avec la lecture des cellules, et ainsi de minimiser les effets de la thermalisation sur l'image acquise. Dans ce cas, la source de chaleur peut être constituée par des pistes conductrices s'étendant le long des lignes du capteur, par exemple des pistes en métal (par exemple en molybdène ou en aluminium), des pistes en un oxyde métallique, éventuellement transparent (par exemple en oxyde d'indium étain), des pistes en silicium polycristallin, ou des pistes en un polymère conducteur.

Dans les capteurs 100 et 120 des figures 1 et 2, les cellules élémentaires d'acquisition 101 et 121 sont des cellules à lecture en charge, c'est-à-dire que la lecture d'une cellule passe par le transfert, sur la piste de sortie CL de la cellule (via le transistor de lecture RS de la cellule), des charges accumulées sur le noeud de lecture SN de la cellule. Un avantage des cellules d'acquisition à lecture en charge est qu'elles sont particulièrement simples et/ou compactes (un seul transistor par cellule dans les exemples des figures 1 et 2). De telles cellules sont particulièrement adaptées aux technologies TFT, par exemple à base de silicium amorphe ou d'oxyde d'indium gallium zinc, dans lesquelles les transistors sont relativement encombrants. A titre d'exemple illustratif et non limitatif, dans les capteurs d'empreintes du type décrit dans la présente demande, le pas des pixels peut typiquement être de l'ordre de 20 à 50 µm, ce qui limite le nombre de transistors que peut comporter chaque cellule.

La figure 3 est un schéma électrique illustrant un autre exemple d'un capteur d'empreintes TFT optique ou thermique 130. Le capteur 130 de la figure 3 comprend des éléments communs avec les capteurs 100 et 120 des figures 1 et 2. Ces éléments ne seront pas décrits à nouveau. Le capteur 130 de la figure 3 diffère des capteurs 100 ou 120 des figures 1 et 2 essentiellement en ce que, dans le capteur 130, des cellules élémentaires d'acquisition 131 à lecture en tension remplacent les cellules à lecture en charge 101 ou 121 des capteurs 100 ou 120. Le capteur 130 diffère en outre des capteurs 100 et 120 en ce que, dans le capteur 130, un ou des étages de sortie 133 remplacent le ou les étages de sortie 103 des capteurs 100 ou 120. Dans le capteur 130, chaque cellule élémentaire d'acquisition 131 comprend un élément de conversion photoélectrique ou pyroélectrique 132 connecté entre un noeud de lecture capacitif SN de la cellule et un noeud d'application d'un potentiel de référence GND de la cellule. A titre d'exemple, l'élément de conversion 132 est un élément de conversion optique PS du type décrit en relation avec la figure 1 ou un élément de conversion thermique PYR du type décrit en relation avec la figure 2. Chaque cellule 131 comprend en outre un transistor de réinitialisation RT reliant son noeud de lecture SN à un noeud d'application d'un potentiel de réinitialisation V_{RT}, par exemple un potentiel positif par rapport au potentiel du noeud GND. Chaque cellule 131 comprend en outre un transistor SF monté en source suiveuse, dont la grille est connectée au noeud SN, et un transistor de lecture RD reliant la source du transistor SF à la piste de sortie CL de la cellule. Le drain du transistor SF est relié à un noeud d'application d'un potentiel de référence, par exemple le potentiel V_{RT} ou un autre potentiel. La grille de commande du transistor RT est connectée à un noeud VG_{RT} d'application d'un potentiel de commande de ce transistor, et la grille du transistor RD est connectée à un noeud VG_{RD} d'application d'un potentiel de commande de ce transistor.

La piste de sortie CL de la cellule 131 est connectée à un étage de sortie 133 du capteur. Dans cet exemple, l'étage de sortie 133 comprend un amplificateur 135 dont une entrée est reliée à la piste CL et dont la sortie est reliée à un convertisseur analogique-numérique 107 (ADC). L'amplificateur 135 est optionnel, et peut notamment être omis si le niveau de potentiel de la piste CL est compatible avec l'entrée du convertisseur analogique-numérique 107.

Le capteur 130 comprend en outre une source lumineuse d'éclairage (non représentée) si les éléments 132 sont des éléments d'acquisition optique, ou une source de chaleur (non représentée) si les éléments 132 sont des éléments d'acquisition pyroélectriques.

Le fonctionnement d'une cellule élémentaire 131 du capteur 130 lors d'une phase d'acquisition d'une empreinte va maintenant être décrit. L'élément de conversion 132 de la cellule est d'abord réinitialisé par l'intermédiaire du transistor RT de la cellule. Le transistor RT est ensuite rendu non passant, et, pendant une période d'intégration, des charges photogénérées ou des charges générées par effet pyroélectrique s'accumulent sur le noeud de lecture SN de la cellule. A la fin de l'intégration, le potentiel du noeud de lecture SN est reporté sur la piste de sortie CL de la cellule par l'intermédiaire des transistors SF et RD. Pour cela, le transistor RD de la cellule est rendu passant. Le potentiel de la piste de sortie CL est alors lu par l'étage de sortie 133 associé à la piste de sortie CL.

Un avantage des cellules d'acquisition à lecture en tension est qu'elles permettent d'obtenir un meilleur rapport signal sur bruit que les cellules à lecture en charge du type décrit en relation avec les figures 1 et 2.

La figure 4 est un schéma électrique illustrant un exemple d'un capteur d'empreintes TFT capacitif 140. Dans l'exemple représenté, le capteur 140 est un capteur à lecture en tension. Le capteur 140 comprend des éléments communs avec le capteur 130 de la figure 3. Ces éléments ne seront pas décrits à nouveau ci-après. Le capteur 140 diffère du capteur 130 de la figure 3 essentiellement en ce que, dans le capteur 140, des cellules élémentaires d'acquisition 141 de type capacitif remplacent les cellules d'acquisition optiques ou thermiques 131 du capteur 130. Dans cet exemple, le capteur 140 ne comprend pas de source lumineuse d'éclairage ni de source de chaleur. Chaque cellule 141 comprend les mêmes éléments qu'une cellule 131 du capteur de la figure 3, à l'exception de l'élément de conversion optique ou pyroélectrique 132. Chaque cellule 141 comprend en outre un condensateur de référence C_{REF}, connecté entre le noeud de lecture SN de la cellule et un noeud CMD d'application d'un signal de commande. Chaque cellule 141 comprend de plus une électrode conductrice EL connectée au noeud SN, l'électrode EL étant revêtue d'une couche diélectrique et étant destinée à former une capacité avec la peau d'un doigt de l'utilisateur. L'électrode EL est de préférence placée au voisinage de la surface supérieure du capteur, de façon que l'épaisseur de diélectrique entre la surface supérieure du capteur (destinée à recevoir le doigt de l'utilisateur) et l'électrode EL ne dépasse pas quelques microns, par exemple 10 µm.

Le fonctionnement du capteur 140 est le suivant. L'utilisateur place un doigt (ou plusieurs) sur ou au-dessus de la surface supérieure du capteur (côté électrodes EL). Lors de l'acquisition d'un point d'image de l'empreinte par une cellule 141, le noeud de lecture SN de la cellule est d'abord réinitialisé par l'intermédiaire du transistor RT de la cellule. Le transistor RT est ensuite rendu non passant, puis un signal de commande, par exemple une tension en créneau ou échelon, est appliqué sur le noeud de commande CMD de la cellule. La capacité de référence C_{REF} et la capacité formée entre l'électrode EL et la peau du doigt forment un pont diviseur capacitif. Un potentiel dépendant de la capacité formée entre l'électrode EL et la peau s'établit alors sur le noeud de lecture SN de la cellule. La valeur de ce potentiel est différente selon que l'électrode EL est surmontée par une crête ou par un creux de la peau de l'utilisateur. Le potentiel du noeud SN est reporté sur la piste de sortie CL de la cellule par l'intermédiaire des transistors SF et RD. Pour cela, le transistor RD de la cellule est rendu passant. Le potentiel de la piste de sortie CL est alors lu par l'étage de sortie 133 associé à la piste de sortie CL. L'échelon appliqué sur le noeud CMD peut alors être ramené à sa valeur initiale.

Selon un aspect de modes de réalisation décrits, on prévoit un capteur d'empreintes réalisé en technologie TFT, ce capteur comportant, sur un substrat de support isolant transparent, par exemple en verre, une pluralité de cellules élémentaires d'acquisition, chaque cellule comportant un photodétecteur, un élément de conversion pyroélectrique, et au moins un transistor TFT. En d'autres termes, on prévoit de combiner, dans chaque cellule élémentaire, un élément d'acquisition optique et un élément d'acquisition thermique, de façon que le capteur fournisse, lors de l'acquisition d'une empreinte digitale ou palmaire, une image optique et une image thermique de l'empreinte.

Un avantage d'un tel capteur est que les deux images sont acquises au moyen d'éléments d'acquisition basés sur des phénomènes n'ayant, à priori, pas de relation physique. En effet, le photodétecteur génère des charges électriques lorsqu'il reçoit des photons, et l'élément pyroélectrique génère des charges électriques lorsque sa température varie. Ceci permet de résoudre, dans une certaine mesure, les difficultés rencontrées lors de l'acquisition d'empreintes de doigts dits "difficiles", c'est-à-dire certains types de doigts sur lesquels les capteurs usuels ne parviennent pas à discriminer de façon satisfaisante les crêtes des creux de la peau. Les inventeurs ont en effet constaté que, généralement, une empreinte difficile à acquérir dans le domaine optique est plus facile à acquérir dans le domaine thermique, et, inversement, une empreinte difficile à acquérir dans le domaine thermique est plus facile à acquérir dans le domaine optique.

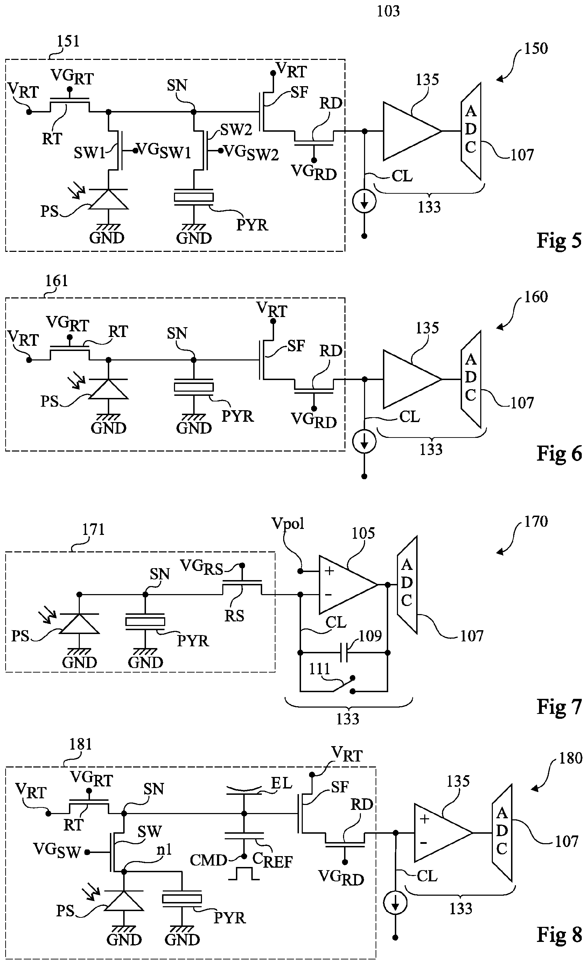

Le fait d'intégrer le photodétecteur et l'élément de conversion pyroélectrique dans une même cellule élémentaire permet de partager des transistors TFT de commande de la cellule entre le photodétecteur et l'élément de conversion pyroélectrique, et ainsi de limiter l'encombrement, la complexité et le coût du capteur. De préférence, comme cela sera détaillé ci-après en relation avec les figures 6, 7, 8, 9 et 10, dans chaque cellule élémentaire, un même transistor TFT de la cellule est connecté à la fois au photodétecteur et à l'élément de conversion pyroélectrique, ce qui permet de limiter l'encombrement. Plus particulièrement, dans les modes de réalisation des figures 6, 7 et 8, dans chaque cellule élémentaire, les éléments de conversion photoélectrique et pyroélectrique sont connectés en parallèle, ce qui permet d'obtenir des cellules élémentaires particulièrement simples et compactes.

La figure 5 est un exemple d'un capteur d'empreintes TFT optique et thermique 150. Le capteur 150 de la figure 5 est un capteur à lecture en tension. Le capteur 150 de la figure 5 comprend des éléments communs avec le capteur 130 de la figure 3. Ces éléments ne seront pas décrits à nouveau. Le capteur 150 de la figure 5 diffère du capteur 130 de la figure 3 essentiellement en ce que, dans le capteur 150, des cellules élémentaires d'acquisition 151, à la fois optiques et thermiques, remplacent les cellules optiques ou thermiques 131 du capteur 130. Dans le capteur 150, chaque cellule élémentaire d'acquisition 151 comprend les mêmes éléments qu'une cellule élémentaire d'acquisition 131 du capteur 130, à l'exception de l'élément de conversion 132. Chaque cellule 151 comprend en outre un photodétecteur PS, par exemple du type décrit en relation avec la figure 1, en série avec un transistor de sélection SW1 entre le noeud capacitif de lecture SN de la cellule et le noeud de référence GND de la cellule. Dans l'exemple représenté, le photodétecteur PS est une photodiode dont l'anode est connectée au noeud GND et dont la cathode est reliée au noeud SN par l'intermédiaire du transistor SW1. Chaque cellule 151 comprend de plus un élément de conversion pyroélectrique PYR, par exemple du type décrit en relation avec la figure 2, en série avec un transistor de sélection SW2 entre le noeud de lecture SN de la cellule et le noeud GND. Dans l'exemple représenté, l'élément pyroélectrique PYR a une première électrode connectée au noeud GND et une deuxième électrode reliée au noeud SN par l'intermédiaire du transistor SW2. La grille du transistor SW1 est reliée à un noeud VG_{SW1} d'application d'un signal de commande de ce transistor, et la grille du transistor SW2 est reliée à un noeud VG_{SW2} d'application d'un signal de commande de ce transistor.

Le capteur 150 comprend en outre une source lumineuse d'éclairage (non représentée) destinée à la mise en oeuvre d'une acquisition optique d'une image, et une source de chaleur (non représentée), distincte de la source lumineuse, destinée à la mise en oeuvre d'une acquisition thermique d'une image.

Comme dans les exemples précédents, les cellules 151 peuvent être disposées en matrice selon des lignes et des colonnes, les cellules d'une même ligne étant commandées simultanément et les cellules de lignes distinctes étant commandées successivement. De plus, comme dans l'exemple de la figure 2, la source de chaleur peut être commandable pour chauffer les cellules 151 ligne par ligne.

Dans l'exemple de la figure 5, les transistors SW1 et SW2 permettent d'isoler le photodétecteur PS et/ou l'élément pyroélectrique PYR du noeud de lecture SN. Deux images, respectivement optique et thermique, peuvent donc être successivement acquises par le capteur. Lors de l'acquisition de l'image optique, les transistors SW1 sont à l'état passant et les transistors SW2 sont non passants. Lors de l'acquisition de l'image thermique, les transistors SW2 sont à l'état passant, et les transistors SW1 sont à l'état non passant. Pour le reste, le capteur peut être commandé de façon identique ou similaire à ce qui a été décrit en relation avec la figure 3. Dans cet exemple, le transistor de réinitialisation RT, et les transistors SF et RD de l'étage de lecture, sont partagés par les deux éléments de conversion PS et PYR. On notera que l'exemple de la figure 5 peut être adapté pour réaliser un capteur optique et thermique à lecture en charges, en remplaçant, dans le capteur de la figure 1, le photodétecteur PS par l'ensemble formé, dans l'exemple de la figure 5, par les transistors SW1 et SW2, le photodétecteur PS, et l'élément pyroélectrique PYR.

Toutefois, un inconvénient du capteur 150 réside dans le surcoût et l'encombrement lié à la présence, dans chaque cellule élémentaire d'acquisition, des deux transistors de sélection SW1 et SW2.

La figure 6 est un schéma électrique illustrant un exemple d'un capteur d'empreintes TFT optique et thermique 160 selon un premier mode de réalisation. Le capteur 160 de la figure 6 est un capteur à lecture en tension. Ce capteur comprend des éléments communs avec le capteur 150 de la figure 5. Ces éléments ne seront pas décrits à nouveau. Le capteur 160 de la figure 6 diffère du capteur 150 de la figure 5 essentiellement en ce que, dans le capteur 160, des cellules élémentaires d'acquisition 161, à la fois optiques et thermiques, remplacent les cellules optiques et thermiques 151 du capteur 150. Les cellules 161 du capteur 160 diffèrent des cellules 151 du capteur 150 en ce qu'elles ne comportent pas les transistors SW1 et SW2 des cellules 151. Plus particulièrement, dans chaque cellule 161, le photodétecteur PS (et plus particulièrement le noeud de cathode de la photodiode PS dans l'exemple représenté) est connecté au noeud SN, et l'élément pyroélectrique PYR est connecté au noeud SN. En d'autres termes, le photodétecteur PS et l'élément pyroélectrique PYR sont connectés en parallèle entre le noeud SN et le noeud GND. Ainsi, dans cet exemple, le photodétecteur PS et l'élément pyroélectrique PYR sont directement connectés au noeud SN, et par conséquent aux transistors RT et SF de la cellule.

Le capteur 160 comprend en outre une source lumineuse d'éclairage (non représentée) destinée à la mise en oeuvre d'une acquisition optique d'une image, et une source de chaleur (non représentée) destinée à la mise en oeuvre d'une acquisition thermique d'une image. La source lumineuse et la source de chaleur sont par exemple commandables, par l'intermédiaire d'un circuit de contrôle non représenté, pour, lors d'une phase d'acquisition d'une empreinte, être alternativement allumées puis éteintes. Ainsi, lors d'une phase d'acquisition d'une image thermique de l'empreinte, la source lumineuse peut être éteinte, et la source de chaleur allumée. Il en résulte que seul l'élément pyroélectrique PYR est susceptible de générer des charges électriques représentatives du motif de l'empreinte à acquérir. Lors d'une phase d'acquisition d'une image optique de l'empreinte, la source lumineuse peut être allumée et la source de chaleur éteinte. Il en résulte que seul le photodétecteur PS est susceptible de générer des charges électriques représentatives du motif de l'empreinte à acquérir. Pour le reste, le capteur 160 peut être commandé de façon identique ou similaire à ce qui a été décrit en relation avec la figure 3.

A titre d'exemple non limitatif, lors de l'acquisition d'une empreinte, la séquence de commande suivante peut être mise en oeuvre :
- réinitialisation du noeud de lecture SN par la mise à l'état passant, puis à l'état non passant, du transistor RT ;
- allumage de la source de chaleur après ouverture du transistor RT, et maintien à l'état éteint de la source lumineuse ;
- extinction de la source de chaleur et lecture du potentiel du noeud SN en vue de la fourniture d'une image thermique de l'empreinte ;
- réinitialisation du noeud de lecture SN par la mise à l'état passant, puis à l'état non passant, du transistor RT ;
- allumage de la source lumineuse après ouverture du transistor RT, et maintien à l'état éteint de la source de chaleur ; et
- lecture du potentiel du noeud SN en vue de la fourniture d'une image optique de l'empreinte (après une période d'intégration) .

On notera que l'ordre d'acquisition des images optique et thermique peut être inversé.

On notera en outre que les niveaux de potentiel de réinitialisation appliqués pour l'acquisition optique et pour l'acquisition thermique ne sont pas nécessairement les mêmes. On peut en particulier prévoir des niveaux de réinitialisation différents pour équilibrer les niveaux des signaux de sortie, et optimiser l'utilisation d'un convertisseur analogique numérique.

On notera de plus que les lectures du signal optique et du signal thermique peuvent être des lectures du type à deux échantillonnages corrélés (généralement désignées dans la technique par l'acronyme CDS), dans lesquelles la valeur de sortie est égale à la différence entre un niveau de signal utile et un niveau de réinitialisation lus sur la piste CL. Ainsi, dans la séquence susmentionnée, ainsi que dans les séquences de commande décrites ci-après, une lecture du niveau de réinitialisation peut être effectuée, via la piste CL, après chaque réinitialisation du noeud de lecture SN.

A titre de variante, lors de l'acquisition de l'image thermique, la source de chaleur peut être allumée avant la fin de la phase de réinitialisation du noeud de lecture (c'est-à-dire avant d'ouvrir le transistor de réinitialisation RT), afin de s'affranchir d'un éventuel bruit parasite lié à des effets transitoires d'appel de courant par la source de chaleur. De plus, lors de l'acquisition de l'image optique, la source lumineuse peut être allumée avant la fin de la phase de réinitialisation du noeud de lecture, afin de s'affranchir d'un éventuel bruit parasite lié à des effets transitoires d'appel de courant par la source lumineuse.

Par ailleurs, on peut tirer profit du fait que, généralement, l'acquisition d'un point d'image thermique par l'élément pyroélectrique PYR est nettement plus rapide que l'acquisition d'un point d'image optique par le photodétecteur PS. A titre d'exemple illustratif et non limitatif, lors d'une acquisition d'une image thermique, la lecture sur le noeud SN d'un potentiel représentatif du motif d'empreinte s'effectue 10 à 500 µs après l'allumage de la source de chaleur (notamment pour s'affranchir d'une éventuelle dégradation de contraste liée à la thermalisation), et, lors d'une acquisition d'une image optique, le temps d'intégration du photodétecteur est compris entre 10 et 50 ms. A titre de variante, on peut donc prévoir de maintenir la source lumineuse de rétroéclairage allumée pendant l'acquisition thermique, et négliger les charges photogénérées produites par le photodétecteur PS pendant l'acquisition thermique. Ceci permet de simplifier la commande de la source lumineuse. De plus, l'acquisition thermique peut être réalisée pendant la période d'intégration optique, ce qui permet de réduire la durée totale nécessaire à l'acquisition des images optique et thermique. A la fin de la période d'intégration optique, les charges générées sur le noeud de lecture SN par l'élément pyroélectrique PYR peuvent en effet être négligées, dans la mesure où la température au début et à la fin de la période d'intégration optique est pratiquement la même. Dans ce cas, l'acquisition thermique est de préférence effectuée au début de la période d'intégration optique, par exemple dans la première moitié de la période d'intégration optique, ou au moins 500 µs avant la fin de la période d'intégration optique, pour s'assurer que les charges générées par l'élément pyroélectrique sur le noeud SN pendant l'acquisition thermique aient le temps de s'annuler par effet thermalisation avant la fin de la période d'intégration optique.

A titre d'exemple non limitatif, lors de l'acquisition d'une empreinte, la séquence de commande suivante peut être mise en oeuvre :
- allumage de la source lumineuse de rétroéclairage (la source lumineuse peut être allumée en permanence) ;
- réinitialisation du noeud de lecture SN par la mise à l'état passant du transistor RT ;
- allumage de la source de chaleur, et mise à l'état non passant du transistor RT ;
- extinction de la source de chaleur (typiquement 20 à 50 µs après son allumage) et lecture du potentiel du noeud SN en vue de la fourniture d'une image thermique de l'empreinte (les charges photogénérées par l'élément PS peuvent être négligées) ;
- poursuite de l'intégration optique sans réinitialisation du noeud de lecture SN (pendant ce temps les autres cellules de la matrice peuvent être lues pour fournir une image thermique) ; et
- lecture du potentiel du noeud SN, typiquement 10 à 50 ms après la réinitialisation de la cellule, en vue de la fourniture d'une image optique de l'empreinte (le signal thermique peut alors être négligé car la température de la cellule est revenue sensiblement à son état initial).

Un avantage du capteur 160 de la figure 6 réside dans le faible nombre de transistors par cellule élémentaire 161 par rapport au capteur de la figure 5.

De préférence, mais non limitativement, les dimensions (surfaces et/ou épaisseurs) du photodétecteur PS et de l'élément pyroélectrique PYR, la puissance de la source lumineuse de rétroéclairage, le temps d'intégration du photodétecteur, et la puissance de la source de chaleur, sont choisis de façon que les niveaux de tension générés sur le noeud SN lors d'une acquisition optique et lors d'une acquisition thermique soient du même ordre de grandeur, ou tout du moins restent dans les deux cas dans une plage de niveaux de tension acceptable pour l'étage de sortie. A titre d'exemple non limitatif, les paramètres ci-dessus sont choisis de façon que, pour un même type de structuration de la peau (crête ou creux) au-dessus d'une cellule élémentaire, le niveau de tension généré sur le noeud SN lors d'une acquisition optique soit compris entre 0,5 et 2 fois le niveau de tension généré sur le noeud SN lors d'une acquisition thermique.

La figure 7 est un schéma électrique illustrant un autre exemple d'un capteur d'empreintes TFT optique et thermique 170 selon le premier mode de réalisation. Le capteur 170 de la figure 7 est un capteur à lecture en charge. Ce capteur comprend des éléments communs avec les capteurs 100 et 120 des figures 1 et 2. Ces éléments ne seront pas décrits à nouveau. Le capteur 170 de la figure 7 diffère des capteurs 100 et 120 des figures 1 et 2 essentiellement en ce que, dans le capteur 170, des cellules élémentaires d'acquisition 171, à la fois optiques et thermiques, remplacent les cellules optiques 101 ou thermiques 121 des capteurs 100 et 120. Les cellules 171 du capteur 170 diffèrent des cellules 101 ou 121 des capteurs 100 et 120 en ce qu'elles comportent un photodétecteur PS et un élément pyroélectrique PYR connectées en parallèle entre les noeuds SN et GND de la cellule. Ainsi, dans cet exemple, le transistor RS de lecture de la cellule est directement connecté à la fois au photodétecteur PS et à l'élément pyroélectrique PYR. Le transistor RS est partagé par le photodétecteur PS et l'élément pyroélectrique PYR.

Le capteur 170 comprend en outre, comme dans l'exemple de la figure 6, une source lumineuse d'éclairage (non représentée) destinée à la mise en oeuvre d'une acquisition optique d'une image, et une source de chaleur (non représentée) destinée à la mise en oeuvre d'une acquisition thermique d'une image. La source lumineuse et la source de chaleur sont par exemple commandables par l'intermédiaire d'un circuit de contrôle non représenté, de façon identique ou similaire à ce qui a été décrit en relation avec la figure 6.

Le fonctionnement du capteur 170 est identique ou similaire à ce qui a été décrit en relation avec la figure 6, à la différence près que, dans le capteur 170, les cellules élémentaires d'acquisition sont des cellules à lecture en charge. En particulier, les réinitialisations des cellules s'effectuent par l'intermédiaire de leurs transistors de lecture RS.

De façon similaire à ce qui a été décrit dans l'exemple de la figure 6, les dimensions (surfaces et/ou épaisseurs) du photodétecteur PS et de l'élément pyroélectrique PYR, la puissance de la source lumineuse de rétroéclairage, le temps d'intégration du photodétecteur, et la puissance de la source de chaleur, sont de préférence choisis de façon que les quantités de charges générées sur le noeud SN lors d'une acquisition optique et lors d'une acquisition thermique soient du même ordre de grandeur.

La figure 8 est un schéma électrique illustrant un autre exemple d'un capteur d'empreintes TFT optique et thermique 180 selon le premier mode de réalisation, ce capteur comportant en outre des moyens d'acquisition de type capacitif. Le capteur 180 de la figure 8 est un capteur à lecture en tension. Ce capteur comprend des éléments communs avec le capteur 160 de la figure 6. Ces éléments ne seront pas décrits à nouveau. Le capteur 180 de la figure 8 diffère du capteur 160 de la figure 6 essentiellement en ce que, dans le capteur 180, des cellules élémentaires d'acquisition 181 remplacent les cellules 161 du capteur 160. Les cellules 181 du capteur diffèrent des cellules 161 du capteur 180 en ce qu'elles comprennent des moyens d'acquisition d'empreinte de type capacitif, par exemple du type décrit en relation avec la figure 4. Plus particulièrement, dans l'exemple représenté, chaque cellule 181 comprend un transistor de sélection SW reliant le noeud de lecture SN de la cellule au photodétecteur PS et à l'élément pyroélectrique PYR. Plus particulièrement, dans chaque cellule 181, le photodétecteur PS et l'élément pyroélectrique PYR sont connectés en parallèle entre le noeud GND et un noeud intermédiaire n1, et le transistor de sélection SW est connecté entre le noeud n1 et le noeud SN. Dans l'exemple représenté, le photodétecteur PS est une photodiode dont l'anode est connectée au noeud GND et dont la cathode est connectée au noeud n1. La grille du transistor de sélection SW est connectée à un noeud VG_{SW} d'application d'un signal de commande de ce transistor. De plus, dans l'exemple représenté, chaque cellule 181 comprend un condensateur de référence C_{REF} connecté entre le noeud de lecture SN de la cellule et un noeud CMD d'application d'un signal de commande, et une électrode conductrice EL connectée au noeud SN, l'électrode EL étant revêtue d'une couche diélectrique et étant destinée à former une capacité avec la peau d'un doigt de l'utilisateur. A titre de variante, le transistor RT peut être connecté entre les noeuds V_{RT} et n1.

Dans l'exemple de la figure 8, le transistor SW permet d'isoler l'étage d'acquisition capacitif de l'étage d'acquisition optique et pyroélectrique. Ceci permet notamment, lors de la mise en oeuvre d'une acquisition capacitive, que les capacités du photodétecteur PS et de l'élément pyroélectrique PYR ne contribuent pas à augmenter la capacité du noeud de lecture SN, ce qui rendrait difficile l'acquisition d'un signal d'empreinte de type capacitif sur le noeud SN.

Dans l'exemple de la figure 8, trois images d'une même empreinte, respectivement optique, thermique et capacitive, peuvent être acquises par le capteur. Le transistor de réinitialisation RT, et les transistors SF et RD de l'étage de lecture, sont partagés par les trois éléments de conversion capacitif, photoélectrique, et pyroélectrique. Lors de l'acquisition de l'image capacitive, les transistors SW des cellules 181 peuvent être mis à l'état non passant, et les cellules peuvent être commandées de façon similaire ou identique à ce qui a été décrit en relation avec la figure 4. Lors de l'acquisition des images thermique et optique, les transistors SW des cellules 181 peuvent être mis à l'état passant, et les cellules peuvent être commandées de façon similaire ou identique à ce qui a été décrit en relation avec la figure 6. On notera que la capacité des éléments PYR et PS est généralement relativement importante par rapport à la capacité du noeud SN (et notamment la capacité C_{REF}). Ainsi, les signaux thermiques et optiques ne sont pas altérés de façon significative par la capacité du noeud SN lorsque le transistor SW est à l'état passant. A titre de variante, l'intégration de la lumière par le photodétecteur PS peut être effectuée en partie ou en totalité pendant la lecture capacitive, ce qui permet de réduire le temps total d'acquisition de l'empreinte. Comme cela a été décrit en relation avec la figure 6, on peut en outre maintenir la source de rétroéclairage pendant toute la durée d'acquisition de l'empreinte, si l'on suppose que le temps de lecture de l'information thermique est négligeable devant le temps d'intégration du photodétecteur.

A titre d'exemple non limitatif, lors de l'acquisition d'une empreinte, la séquence de commande suivante peut être mise en oeuvre :
- réinitialisation du noeud de lecture SN par la mise à l'état passant du transistor RT, et réinitialisation du noeud n1 par la mise à l'état passant du transistor SW ;
- ouverture du transistor de sélection SW pour isoler l'étage d'acquisition optique et thermique du reste de la cellule (l'ouverture du transistor SW marque le début de la période d'intégration de la lumière par le photodétecteur PS) ;
- ouverture du transistor de réinitialisation RT pour isoler le noeud de lecture SN du noeud V_{RT}, et lecture de la valeur de référence pour la lecture capacitive ;
- lecture capacitive de l'empreinte ;
- allumage de la source de chaleur avant la fin de la lecture capacitive ;
- à l'issue de la lecture capacitive, réinitialisation optionnelle du noeud de lecture SN par fermeture puis réouverture du transistor RT ;
- fermeture du transistor de sélection SW et lecture de la valeur de référence pour les lectures thermique et optique ;
- extinction de la source de chaleur et lecture du potentiel du noeud SN en vue de la fourniture d'une image thermique de l'empreinte (le signal optique peut être négligé car les durées des acquisitions capacitive et thermique sont relativement brèves par rapport au temps d'intégration de l'acquisition optique) ;
- poursuite de l'intégration optique sans réinitialisation du noeud de lecture SN (pendant ce temps les autres cellules de la matrice peuvent être lues) ; et
- lecture du potentiel du noeud SN à la fin de la période d'intégration du photodétecteur, en vue de la fourniture d'une image optique (le signal thermique peut alors être négligé car la température de la cellule est revenue sensiblement à son état initial).

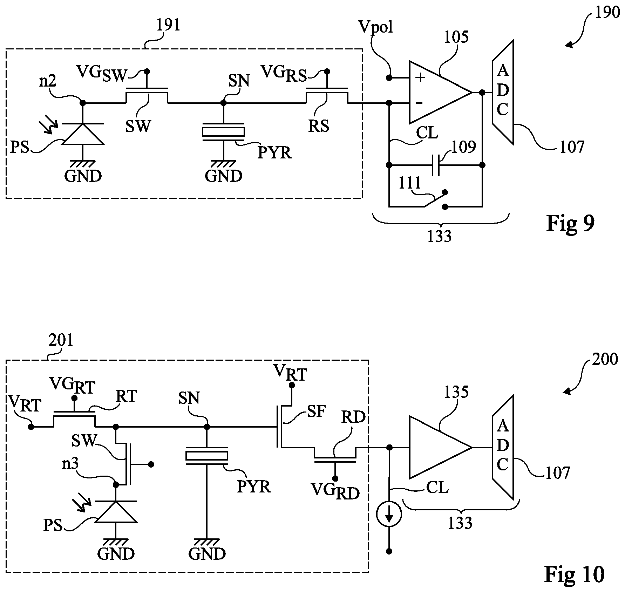

La figure 9 est un schéma électrique illustrant un exemple d'un capteur d'empreintes TFT optique et thermique 190 selon un deuxième mode de réalisation. Le capteur 190 de la figure 9 est un capteur à lecture en charge. Ce capteur comprend des éléments communs avec le capteur 170 de la figure 7. Ces éléments ne seront pas décrits à nouveau. Le capteur 190 de la figure 9 diffère du capteur 170 de la figure 7 essentiellement en ce que, dans le capteur 190, des cellules élémentaires d'acquisition 191 replacent les cellules 171 du capteur 170. Les cellules 191 du capteur 190 diffèrent des cellules 171 du capteur 170 en ce que, dans les cellules 191, le photodétecteur PS n'est pas connecté directement entre les noeuds GND et SN, mais est connecté entre le noeud GND et un noeud intermédiaire n2, le noeud n2 étant relié au noeud SN par un transistor de sélection SW. L'élément pyroélectrique PYR est quant à lui connecté directement au noeud SN. Plus particulièrement, dans cet exemple, le photodétecteur PS est une photodiode dont l'anode est connectée au noeud GND et dont la cathode est connectée au noeud n2. Ainsi, le transistor de sélection SW est connecté à la fois au photodétecteur PS et à l'élément pyroélectrique PYR. Le transistor de lecture RS de la cellule est partagé par le photodétecteur PS et l'élément pyroélectrique PYR.

Dans le capteur 190, les transistors SW permettent, dans chaque cellule élémentaire d'acquisition, d'isoler le photodétecteur PS du reste de la cellule.

Un avantage est que ceci offre des possibilités de commande supplémentaires par rapport au capteur 170 de la figure 7, tout en limitant le coût et l'encombrement par rapport à un capteur à deux transistors de sélection SW1 et SW2 par cellule, tel que décrit en relation avec la figure 5. De plus, ceci donne plus de flexibilité quant au dimensionnement des éléments de conversion optique PS et thermique PYR, que dans un capteur du type décrit en relation avec la figure 7.

A titre d'exemple non limitatif, lors de l'acquisition d'une empreinte, la séquence de commande suivante peut être mise en oeuvre :
- réinitialisation du noeud de lecture SN et du noeud intermédiaire n2 par la mise à l'état passant des transistors RS et SW ;
- ouverture du transistor de sélection SW pour isoler le photodétecteur du noeud SN, et ouverture du transistor RS pour isoler le noeud SN de la piste de sortie CL ;
- allumage de la source de chaleur et de la source d'éclairage (à noter que la source d'éclairage peut en pratique rester allumée en permanence, le photodétecteur PS étant isolé de l'élément pyroélectrique) ;
- extinction de la source de chaleur et lecture du potentiel du noeud SN en vue de la fourniture d'une image thermique de l'empreinte ;
- poursuite de l'intégration optique (pendant ce temps les autres cellules de la matrice peuvent être lues pour fournir l'image thermique) ; et
- fermeture du transistor SW et lecture du potentiel du noeud SN à la fin de la période d'intégration du photodétecteur, en vue de la fourniture d'une image optique.

La figure 10 est un schéma électrique illustrant un autre exemple d'un capteur d'empreintes TFT optique et thermique 200 selon le deuxième mode de réalisation. Le capteur 200 de la figure 10 est un capteur à lecture en tension. Ce capteur comprend des éléments communs avec le capteur 160 de la figure 6. Ces éléments ne seront pas décrits à nouveau. Le capteur 200 de la figure 10 diffère du capteur 160 de la figure 6 essentiellement en ce que, dans le capteur 200, des cellules élémentaires d'acquisition 201 remplacent les cellules 161 du capteur 160. Les cellules 201 du capteur 200 diffèrent des cellules 161 du capteur 160 en ce que, dans les cellules 201, le photodétecteur PS n'est pas connecté directement entre les noeuds GND et SN, mais est connecté entre le noeud GND et un noeud intermédiaire n3, le noeud n3 étant relié au noeud SN par un transistor de sélection SW. L'élément pyroélectrique PYR est quant à lui connecté directement au noeud SN. Plus particulièrement, dans cet exemple, le photodétecteur PS est une photodiode dont l'anode est connectée au noeud GND et dont la cathode est connectée au noeud n3. Ainsi, le transistor de sélection SW est connecté à la fois au photodétecteur PS et à l'élément pyroélectrique PYR. Les transistors RT, SF et RD de la cellule sont partagés par le photodétecteur PS et l'élément pyroélectrique PYR.

Dans le capteur 200, les transistors SW permettent, dans chaque cellule élémentaire d'acquisition, d'isoler le photodétecteur PS du reste de la cellule, ce qui offre des possibilités de commande supplémentaires par rapport au capteur 160 de la figure 6, tout limitant le coût et l'encombrement par rapport à un capteur à deux transistors de sélection SW1 et SW2 par cellule, du type décrit en relation avec la figure 5. De plus, ceci donne plus de flexibilité quant au dimensionnement des éléments de conversion optique PS et thermique PYR, que dans un capteur du type décrit en relation avec la figure 6.

A titre d'exemple non limitatif, lors de l'acquisition d'une empreinte, la séquence de commande suivante peut être mise en oeuvre :
- allumage de la source lumineuse d'éclairage (dans cet exemple, la source lumineuse peut rester allumée en permanence) ;
- réinitialisation du noeud de lecture SN par la mise à l'état passant du transistor RT, puis ouverture du transistor RT ;
- fermeture du transistor SW - les charges photogénérées par le photodétecteur PS depuis sa précédente réinitialisation sont alors réparties sur l'ensemble de la capacité du noeud de lecture SN ;
- lecture du potentiel du noeud SN à la fin de la période d'intégration du photodétecteur, en vue de la fourniture d'une image optique.
- réinitialisation des noeuds n3 et SN par la mise à l'état passant des transistors RT et SW ;
- ouverture du transistor SW, pour isoler le photodétecteur PS du noeud SN (ce qui définit le début d'une nouvelle période d'intégration du photodétecteur PS) ;
- ouverture du transistor de réinitialisation RT ;
- allumage de la source de chaleur ;
- extinction de la source de chaleur et lecture du potentiel du noeud SN en vue de la fourniture d'une image thermique de l'empreinte.

A titre de variante, dans les exemples des figures 9 et 10, le photodétecteur PS et l'élément pyroélectrique PYR peuvent être intervertis, c'est-à-dire que l'élément PYR peut être placé derrière le transistor de sélection SW, entre le noeud n2 ou n3 et le noeud GND, le photodétecteur PS étant alors directement connecté au noeud SN. Ce choix peut être effectué en fonction des valeurs respectives des capacités des éléments PYR et PS.

Des modes de réalisation particuliers ont été décrits. Diverses variantes et modifications apparaîtront à l'homme de l'art.

En particulier, on notera que, dans un capteur d'empreinte optique, le photodétecteur peut générer, en plus du signal utile représentatif du motif de l'empreinte à acquérir, du bruit lié notamment aux courants d'obscurité et/ou à la lumière ambiante. Pour s'affranchir de ces sources de bruit, on peut prévoir, dans les modes de réalisation susmentionnés, d'effectuer une lecture à vide, c'est-à-dire sans doigt posé sur le capteur et sans allumer la source de rétroéclairage ni la source de lumière, de façon à acquérir une image représentative du bruit généré par le photodétecteur PS (lié notamment aux courants d'obscurité et à la lumière ambiante parasite). Une fois cette image acquise, elle peut être soustraite à l'image optique finale, et, si le photodétecteur n'est pas isolé de l'élément pyroélectrique, à l'image thermique finale.

Par ailleurs, au lieu d'effectuer deux lectures distinctes, respectivement thermique et optique, en cherchant à distinguer le signal thermique du signal optique, comme cela a été décrit ci-dessus, on peut prévoir de lire la somme des charges photogénérées et des charges générées par l'élément pyroélectrique, sans chercher à les séparer, afin d'acquérir une image combinant le signal optique et le signal thermique, ce qui peut s'avérer utile pour acquérir certaines empreintes difficiles. A titre d'exemple, dans les modes de réalisation des figures 6, 7 et 8, on peut prévoir d'allumer d'abord la source lumineuse, puis, vers la fin de la période d'intégration optique, d'allumer la source de chaleur de façon que l'élément pyroélectrique génère des charges sur le noeud SN, puis de lire sur le noeud SN une valeur représentative de la somme du signal optique et du signal thermique.

En outre, bien que l'on ait décrit ci-dessus des exemples de procédé de commande dans lesquels, lors de l'acquisition d'une empreinte, une image optique et une image thermique sont lues, on pourra prévoir des variantes de réalisation dans lesquelles on lit plusieurs images optiques et/ou plusieurs images thermiques de la même empreinte. A titre d'exemple, si la période d'intégration optique est plusieurs fois supérieure au temps nécessaire à la mise en oeuvre des étapes de lecture thermique de l'ensemble des lignes du capteur, on peut prévoir de balayer successivement plusieurs fois l'ensemble du capteur pour acquérir plusieurs images thermiques, puis, au dernier balayage du capteur, d'acquérir l'image optique.

De plus, dans les modes de réalisation des figures 9 et 10, le transistor de sélection SW entre le photodétecteur PS et l'élément pyroélectrique PYR peut avantageusement être utilisé pour mettre en oeuvre une séquence de lecture de type "global shutter" (ou lecture à obturation globale), c'est-à-dire dans laquelle, lors d'une acquisition d'une image optique, tous les photodétecteurs PS du capteur sont intégrés simultanément. Dans chaque cellule élémentaire d'acquisition, l'élément pyroélectrique PYR peut alors servir d'élément de mémorisation pour stocker la valeur acquise par le photodétecteur, en attendant sa lecture. Ce mode de réalisation permet avantageusement d'utiliser un temps d'intégration faible devant le temps de lecture de l'ensemble de la matrice, la source lumineuse étant flashée de manière synchrone avec l'acquisition. A titre d'exemple non limitatif, lors d'une acquisition d'une empreinte, la séquence de commande suivante peut être mise en oeuvre :
- mise à l'état passant des transistors SW de l'ensemble des cellules du capteur ;
- réinitialisation de l'ensemble des cellules du capteur, puis mise à l'état non passant des transistors SW ;
- allumage de la source lumineuse pendant la période d'intégration optique du capteur ;
- à la fin de la période d'intégration, fermeture des transistors de sélection SW dans l'ensemble des cellules du capteur (des charges photogénérées pendant l'intégration sont alors transférées sur le noeud SN, dans la capacité formée par l'élément pyroélectrique) puis ouverture des transistors de sélection SW (la tension aux bornes de la photodiode peut alors continuer à évoluer indépendamment du noeud SN) ;
- lecture, par exemple ligne par ligne, dans l'ensemble des cellules du capteur, du niveau de signal mémorisé sur le noeud SN en vue de la fourniture de l'image optique ;
- dans chaque cellule, après la lecture du signal optique, réinitialisation du noeud SN en fermant le transistor RT et en laissant le transistor SW ouvert ;
- allumage de la source de chaleur ;
- extinction de la source de chaleur et lecture du signal sur le noeud SN en vue de la fourniture d'une image thermique.

Par ailleurs, les modes de réalisation décrits ci-dessus peuvent être combinés avec d'autres technologies d'acquisition d'empreintes digitales, par exemple des technologies d'acquisition à ultrasons ou à champ électrique, ou des technologies dans lesquelles un signal RF est appliqué à travers la peau de l'utilisateur.

En outre, l'homme de l'art saura adapter les modes de réalisation décrits pour réaliser des capteurs mettant en oeuvre des acquisitions d'images thermiques de type passif, c'est-à-dire des capteurs sans source de chaleur active. Dans ce cas, la variation de température exploitée par les éléments de conversion pyroélectrique PYR est une variation qui se produit lorsque l'utilisateur pose son doigt sur le capteur, et/ou le retire du capteur. De tels capteurs peuvent être de type statique (la surface du capteur est au moins égale à la surface de l'empreinte à acquérir, et le doigt ne bouge pas par rapport au capteur pendant l'acquisition) ou de type à balayage (la surface du capteur est inférieure à la surface de l'empreinte à acquérir, et le doigt défile devant le capteur lors de l'acquisition).

## Revendications

1. Capteur d'empreintes digitales ou palmaires (150 ; 160 ; 170 ; 180 ; 190 ; 200) comportant :
sur un substrat de support, une pluralité de cellules élémentaires d'acquisition (151 ; 161 ; 171 ; 181 ; 191 ; 201) réalisées en technologie TFT, chaque cellule comportant un photodétecteur (PS) et un élément de conversion pyroélectrique (PYR) reliés à un même noeud capacitif de lecture (SN) de la cellule, et un circuit de lecture (RS ; SF, RD) reliant le noeud capacitif de lecture (SN) de la cellule à une piste conductrice (CL) de sortie de la cellule ;
une source lumineuse d'éclairage ; et
une source de chaleur distincte de la source lumineuse.

2. Capteur (150 ; 160 ; 170 ; 180 ; 190 ; 200) selon la revendication 1, dans lequel le substrat de support est transparent.

3. Capteur (160 ; 170 ; 180) selon la revendication 1 ou 2, dans lequel le photodétecteur (PS) et l'élément de conversion pyroélectrique (PYR) sont connectés en parallèle.

4. Capteur (160 ; 170 ; 180) selon l'une quelconque des revendications 1 à 3, comportant un circuit adapté à commander l'acquisition d'une image thermique et l'acquisition d'une image optique par les cellules (161 ; 171 ; 181) du capteur, ce circuit étant adapté, pendant toute la phase d'acquisition de l'image optique, à allumer la source lumineuse et maintenir la source de chaleur éteinte, et, pendant toute la phase d'acquisition de l'image thermique, à maintenir la source lumineuse éteinte.

5. Capteur (160 ; 170 ; 180) selon l'une quelconque des revendications 1 ou 3, comportant un circuit adapté à commander l'acquisition d'une image thermique et l'acquisition d'une image optique par les cellules (161 ; 171 ; 181) du capteur, ce circuit étant adapté à mettre en oeuvre l'acquisition de l'image thermique pendant une partie d'une période d'intégration de la phase d'acquisition de l'image optique pendant laquelle la source lumineuse est allumée.

6. Capteur (160 ; 170) selon l'une quelconque des revendications 1 à 5, dans lequel, dans chaque cellule (161 ; 171), le photodétecteur (PS) et l'élément de conversion pyroélectrique (PYR) sont connectés au noeud capacitif de lecture (SN) de la cellule.

7. Capteur (180) selon l'une quelconque des revendications 1 à 5, dans lequel, dans chaque cellule (181), le photodétecteur (PS) et l'élément de conversion pyroélectrique (PYR) sont connectés à un noeud intermédiaire (n1) de la cellule, le noeud intermédiaire étant relié au noeud capacitif de lecture (SN) de la cellule par un transistor de sélection (SW), et chaque cellule (181) comprenant en outre :
un condensateur de référence (C_{REF}) connecté entre le noeud capacitif de lecture (SN) et un noeud (CMD) d'application d'un signal de commande ; et
une électrode (EL) connectée au noeud capacitif de lecture (SN), l'électrode (EL) étant revêtue d'une couche diélectrique et étant destinée à former une capacité avec la peau d'un utilisateur en vue d'une acquisition capacitive d'une image d'empreinte.

8. Capteur (190 ; 200) selon la revendication 1 ou 2, dans lequel le photodétecteur (PS) et l'élément de conversion pyroélectrique (PYR) sont connectés à un même transistor de sélection (SW) permettant, à l'état ouvert, d'isoler le photodétecteur (PS) de l'élément pyroélectrique (PYR).

9. Capteur (150 ; 160 ; 170 ; 180 ; 190 ; 200) selon l'une quelconque des revendications 1 à 8, dans lequel les cellules (151 ; 161 ; 171 ; 181 ; 191 ; 201) sont disposées en matrice selon des lignes et des colonnes, et dans lequel la source de chaleur est commandable pour chauffer les cellules (151 ; 161 ; 171 ; 181 ; 191 ; 201) ligne par ligne.

10. Capteur (150 ; 160 ; 180 ; 200) selon l'une quelconque des revendications 1 à 9, dans lequel les cellules (151 ; 161 ; 181 ; 201) sont des cellules à lecture en tension, chaque cellule comportant un transistor de réinitialisation (RT) reliant le noeud capacitif de lecture (SN) à un noeud (V_{RT}) d'application d'un potentiel de réinitialisation, et le circuit de lecture de chaque cellule comportant un transistor (SF) monté en source suiveuse, dont la grille est connectée au noeud de lecture (SN), et dont la source est reliée à une piste de sortie (CL) de la cellule par l'intermédiaire d'un transistor de lecture (RD) .

11. Capteur (170 ; 190) selon l'une quelconque des revendications 1 à 9, dans lequel les cellules (171 ; 191) sont des cellules à lecture en charge, le circuit de lecture de chaque cellule comportant un transistor de lecture (RS) reliant le noeud de lecture (SN) à une piste de sortie (CL) de la cellule.

## Patentansprüche

1. Ein Finger- oder Handabdrucksensor (150; 160; 170; 180; 190; 200), der Folgendes aufweist:
auf einem Trägersubstrat eine Vielzahl von elementaren Erfassungszellen (151; 161; 171; 181; 191; 201), die in TFT-Technologie realisiert sind, wobei jede Zelle einen Fotodetektor (PS) und ein pyroelektrisches Umwandlungselement (PYR) aufweist, das an einen gleichen kapazitiven Sensorknoten (SN) der Zelle gekoppelt ist, und eine Ausleseschaltung (RS; SF, RD), die den kapazitiven Sensorknoten (SN) der Zelle an eine Ausgangsleiterbahn (CL) der Zelle koppelt;
eine Beleuchtungslichtquelle; und
eine Wärmequelle, die sich von der Lichtquelle unterscheidet.

2. Sensor (150; 160; 170; 180; 190; 200) nach Anspruch 1, wobei das Trägersubstrat transparent ist.

3. Sensor (160; 170; 180) nach Anspruch 1 oder 2, wobei der Fotodetektor (PS) und das pyroelektrische Umwandlungselement (PYR) parallel geschaltet sind.

4. Sensor (160; 170; 180) nach einem der Ansprüche 1 bis 3, der eine Schaltung aufweist, die geeignet ist zum Steuern der Erfassung eines thermischen Bildes und der Erfassung eines optischen Bildes durch die Sensorzellen (161; 171; 181), wobei die Schaltung geeignet ist, während der gesamten optischen Bilderfassungsphase die Lichtquelle einzuschalten und die Wärmequelle ausgeschaltet zu halten und während der gesamten thermischen Bilderfassungsphase die Lichtquelle ausgeschaltet zu halten.

5. Sensor (160; 170; 180) nach einem der Ansprüche 1 bis 3, der eine Schaltung aufweist, die geeignet ist zum Steuern der Erfassung eines thermischen Bildes und der Erfassung eines optischen Bildes durch die Sensorzellen (161; 171; 181), wobei die Schaltung geeignet ist zum Implementieren der Erfassung des thermischen Bildes während einem Teil einer Integrationsdauer der optischen Bilderfassungsphase während der die Lichtquelle an ist.

6. Sensor (160; 170) nach einem der Ansprüche 1 bis 5, wobei in jeder Zelle (161; 171) der Fotodetektor (PS) und das pyroelektrische Umwandlungselement (PYR) an den kapazitiven Sensorknoten (SN) der Zelle gekoppelt sind.

7. Sensor (180) nach einem der Ansprüche 1 bis 5, wobei in jeder Zelle (181) der Fotodetektor (PS) und das pyroelektrische Umwandlungselement (PYR) an einen Zwischenknoten (n1) der Zelle gekoppelt sind, wobei der Zwischenknoten an den kapazitiven Sensorknoten (SN) der Zelle durch einen Auswahltransistor (SW) gekoppelt ist und jede Zelle (181) ferner Folgendes aufweist:
einen Referenzkondensator (C_{REF}), der zwischen dem kapazitiven Sensorknoten (SN) und einem Knoten (CMD) des Anlegens eines Kontrollsignals verbunden ist; und
eine Elektrode (EL), die mit dem kapazitiven Sensorknoten (SN) verbunden ist, wobei die Elektrode (EL) mit einer dielektrischen Schicht beschichtet ist und bestimmt ist zum Bilden einer Kapazität mit der Haut eines Nutzers zur kapazitiven Erfassung eines Abdruckbildes.

8. Sensor (190; 200) nach Anspruch 1 oder 2, wobei der Fotodetektor (PS) und das pyroelektrische Umwandlungselement (PYR) mit einem gleichen Selektionstransistor (SW) verbunden sind, um in dem Auszustand zu ermöglichen, den Fotodetektor (PS) von dem pyroelektrischen Element (PYR) zu isolieren.

9. Sensor (150; 160; 170; 180; 190; 200) nach einem der Ansprüche 1 bis 8, wobei die Zellen (151; 161; 171; 181; 191; 201) in einer Reihen- und Spaltenanordnung angeordnet sind und wobei die Wärmequelle zum Erwärmen der Zellen (151; 161; 171; 181; 191; 201) Reihe für Reihe steuerbar ist.

10. Sensor (150; 160; 180; 200) nach einem der Ansprüche 1 bis 9, wobei die Zellen (151; 161; 181; 201) Spannungsauslesezellen sind, wobei jede Zelle einen Reset-Transistor (RT) aufweist, der den kapazitiven Sensorknoten (SN) an einen Knoten (V_{RT}) des Anlegens eines Reset-Potentials koppelt und die Ausleseschaltung jeder Zelle einen Transistor (SF), der als eine Folgequelle angeordnet ist, aufweist, dessen Gatter mit dem Sensorknoten (SN) verbunden ist und dessen Quelle mit einer Ausgangsbahn (CL) der Zelle über einen Auslesetransistor (RD) verbunden ist.

11. Sensor (170; 190) nach einem der Ansprüche 1 bis 9, wobei die Zellen (171; 191) Ladeauslesezellen sind, wobei die Ausleseschaltung jeder Zelle einen Auslesetransistor (RS) aufweist, der den Sensorknoten (SN) an die Ausgangsbahn (CL) jeder Zelle koppelt.

## Claims

1. A fingerprint or palmprint sensor (150; 160; 170; 180; 190; 200) comprising:
on a support substrate, a plurality of elementary acquisition cells (151; 161; 171; 181; 191; 201) realized in TFT technology, each cell comprising a photodetector (PS) and a pyroelectric conversion element (PYR) coupled to a same capacitive sense node (SN) of the cell, and a readout circuit (RS; SF, RD) coupling the capacitive sense node (SN) of the cell to an output conductive track (CL) of the cell;
an illumination light source; and
a heat source distinct from the light source.

2. The sensor (150; 160; 170; 180; 190; 200) of claim 1, wherein the support substrate is transparent.

3. The sensor (160; 170; 180) of claim 1 or 2, wherein the photodetector (PS) and the pyroelectric conversion element (PYR) are connected in parallel.

4. The sensor (160; 170; 180) of any of claims 1 to 3, comprising a circuit capable of controlling the acquisition of a thermal image and the acquisition of an optical image by the sensor cells (161; 171; 181), the circuit being capable, during the entire optical image acquisition phase, of turning on the light source and of keeping the heat source off and, during the entire thermal image acquisition phase, of keeping the light source off.

5. The sensor (160; 170; 180) of any of claims 1 to 3, comprising a circuit capable of controlling the acquisition of a thermal image and the acquisition of an optical image by the sensor cells (161; 171; 181), the circuit being capable of implementing the acquisition of the thermal image during part of an integration period of the optical image acquisition phase during which the light source is on.

6. The sensor (160; 170) of any of claims 1 to 5, wherein, in each cell (161; 171), the photodetector (PS) and the pyroelectric conversion element (PYR) are connected to the capacitive sense node (SN) of the cell.

7. The sensor (180) of any of claims 1 to 5, wherein, in each cell (181), the photodetector (PS) and the pyroelectric conversion element (PYR) are connected to an intermediate node (n1) of the cell, the intermediate node being coupled to the capacitive sense node (SN) of the cell by a selection transistor (SW), and each cell (181) further comprising:
a reference capacitor (C_{REF}) connected between the capacitive sense node (SN) and a node (CMD) of application of a control signal; and
an electrode (EL) connected to the capacitive sense node (SN), the electrode (EL) being coated with a dielectric layer and being intended to form a capacitance with a user's skin for a capacitive acquisition of a print image.

8. The sensor (190; 200) of claim 1 or 2, wherein the photodetector (PS) and the pyroelectric conversion element (PYR) are connected to a same selection transistor (SW) enabling, in the off state, to isolate the photodetector (PS) from the pyroelectric element (PYR).

9. The sensor (150; 160; 170; 180; 190; 200) of any of claims 1 to 8, wherein the cells (151; 161; 171; 181; 191; 201) are arranged in an array of rows and columns, and wherein the heat source is controllable to heat the cells (151; 161; 171; 181; 191; 201) row by row.

10. The sensor (150; 160; 180; 200) of any of claims 1 to 9, wherein the cells (151; 161; 181; 201) are voltage readout cells, each cell comprising a reset transistor (RT) coupling the capacitive sense node (SN) to a node (V_{RT}) of application of a reset potential, and the readout circuit of each cell comprising a transistor (SF) assembled as a follower source, having its gate connected to the sense node (SN), and having its source coupled to an output track (CL) of the cell via a readout transistor (RD).

11. The sensor (170; 190) of any of claims 1 to 9, wherein the cells (171; 191) are charge readout cells, the readout circuit of each cell comprising a readout transistor (RS) coupling the sense node (SN) to an output track (CL) of the cell.
